# FASCICULE DE BREVET EUROPEEN

(11) **EP 1 282 431 B1**
(45) Date de publication et mention de la délivrance du brevet: **18.02.2004**
(21) Numéro de dépôt: 00929643.5
(22) Date de dépôt: 19.05.2000
(51) Int. Cl.: A61K 35/78, A61P 9/00

(54) **COMPOSITION PHYTOTHERAPEUTIQUE PREVENTIVE DES MALADIES CARDIO-VASCULAIRES**
PRÄVENTIVES PHYTOTHERAPEUTIKUM FÜR KARDIOVASKULÄREN ERKRANKUNGEN
PHYTOTHERAPEUTIC COMPOSITION FOR PREVENTING CARDIOVASCULAR DISEASES

(43) Date de publication de la demande: 12.02.2003
(73) Titulaire: Dervieux, Dominique, Naples, FL 34119 (US)
(72) Inventeur: Dervieux, Dominique, Naples, FL 34119 (US)
(74) Mandataire: Bonneau, Gérard
(86) Numéro de dépôt international: PCT/FR2000/001372
(87) Numéro de publication internationale: WO 2001/087318

(56) Documents cités:
- DE-A- 19 720 767
- DATABASE WPI Section Ch, Week 200034 Derwent Publications Ltd., London, GB; Class B05, AN 2000-388009 XP002160494 & BR 9 802 636 A (MOREIRA M C), 21 mars 2000 (2000-03-21)

## Description

### Domaine technique :

La présente invention concerne les compositions phytothérapeutiques et plus particulièrement une composition phytothérapeutique permettant de prévenir les maladies cardio-vasculaires, des thromboses vasculaires et plus généralement les effets du vieillissement.

### Etat de la technique :

Durant les vingt dernières années, l'espérance de vie a fortement progressé. En effet, la proportion de personnes âgées connaît un accroissement important. Mais ce vieillissement de la population s'accompagne de problèmes de santé qui sont également liés à notre mode de vie. En effet, de façon générale dans les pays industrialisés, la diminution de l'activité physique couplée à une modification du régime alimentaire entraînant un déséquilibre nutritionnel sont parmi les facteurs à l'origine de l'augmentation des maladies cardio-vasculaires. Ces problèmes sont principalement l'hypercholestérolémie, l'hypertension artérielle et l'athérosclérose. Ces problèmes qui apparaissent généralement après soixante ans, sont étroitement liés les uns aux autres. En effet, lorsque le taux de cholestérol est important, celui-ci se retrouve dans le sang lié à des lipoprotéines LDL (Low Density Lipoproteins). Il peut être également absorbé par les macrophages, cellules du système immunitaire. Ces LDL et macrophages se retrouvent dans la circulation sanguine et viennent s'agréger sur la paroi des vaisseaux avec notamment des plaquettes, induisant un rétrécissement du vaisseau. Ces agrégats sont appelés plaques d'athérome. Les patients souffrent alors d'athérosclérose. Ces plaques d'athérome se développent jusqu'à obturation complète du vaisseau. Selon la localisation du vaisseau obturé, les patients peuvent être victimes d'accidents vasculaires cardiaques ou cérébraux, tels que des infarctus et des thromboses vasculaires (artérielles ou veineuses) et mourir. De plus, si une plaque est détachée d'un vaisseau, elle peut être envoyée vers le cerveau et être arrêtée par une artère ayant la même taille, entraînant une embolie cérébrale. L'hypertension artérielle est un facteur aggravant de l'athérosclérose. La sédentarité, le stress et les déplacements en avion sont des facteurs aggravant du risque de thrombose.

Il existe des traitements curatifs de l'athérosclérose, ayant pour but d'éviter ces conséquences graves. Ces traitements sont lourds et le suivi médical des patients est soutenu. Ces traitements consistent notamment à faire baisser le taux de cholestérol dans le sang, à normaliser la pression artérielle.

Cependant, il n'existe pas de traitements préventifs aux problèmes cardio-vasculaires tels que l'athérosclérose et les thromboses vasculaires, si ce n'est une modification du régime alimentaire ayant pour but de diminuer l'apport de graisse et des anticoagulants lorsque le risque est majeur.

### Exposé de l'invention :

Le but de l'invention est de pallier ces inconvénients en fournissant une composition phytothérapeutique ayant pour but de prévenir les problèmes cardiovasculaires et plus particulièrement l'athérosclérose, ainsi que les thromboses vasculaires.

L'invention concerne donc une composition phytothérapeutique ayant une action préventive des maladies cardio-vasculaires, des thromboses vasculaires et plus généralement une action anti-vieillissement comprenant un extrait pris dans le groupe composé de marc de raisin après vinification, d'extrait de vin, d'extrait de pépin de raisin et au moins un extrait d'un végétal riche en salicylés.

Selon un mode de réalisation préféré, la composition phytothérapeutique selon l'invention comprend du marc de raisin récupéré en fond de cuve après vinification et un extrait de reine des prés (*Filipendula ulmaria*).

### Description de l'invention :

Les buts, objets et caractéristiques de l'invention ressortiront mieux à la lecture de la description non limitative qui suit.

Depuis plusieurs années, des études réalisées ont permis de mettre en évidence le rôle bénéfique du vin rouge sur la santé. Il a été clairement prouvé, qu'à facteurs de risques égaux, les Français ont moins de maladies cardiovasculaires que la plupart des occidentaux. Cet effet est dû aux composés qui sont présents dans le vin et en particulier les composés anti-oxydants tels que les polyphénols et les procyanidols (OPC). Ces antioxydants, et spécialement les polyphénols tels que le resvératrol, empêchent l'oxydation des LDL. Les LDL amènent le cholestérol alimentaire du foie vers les cellules de l'organisme par l'intermédiaire de la circulation sanguine. A l'inverse, les HDL (High Density Lipoproteins) récupèrent le cholestérol en excès et le ramènent au foie pour permettre son élimination. Pour que les LDL approvisionnent les cellules, elles doivent se fixer sur des récepteurs extracellulaires spécifiques. Lorsque la cellule dispose de suffisamment de cholestérol, elle cesse de synthétiser ces récepteurs. Lorsqu'il y a trop de LDL circulantes (dues par exemple à une alimentation déséquilibrée), celles-ci s'oxydent. Elles ne sont alors plus reconnues par le récepteur spécifique sous contrôle cellulaire mais par un autre récepteur dont la synthèse n'est pas régulée. Les cellules se remplissent alors de cholestérol. Les premières victimes de ce phénomène sont les macrophages. Remplis de cholestérol, ces derniers s'infiltrent dans les parois de vaisseaux et favorisent la formation des plaques d'athérome, par agrégation des plaquettes et autres constituants du sang.

Les polyphénols contenus dans différents extraits tels que l'extrait de marc de raisin après fermentation, l'extrait de vin rouge ou l'extrait de pépins de raisin, et particulièrement le resvératrol, empêchent l'oxydation des LDL et donc permettent de diminuer la formation des plaques d'athérome dans les vaisseaux. Les procyanidols (OPC) ont une action de renforcement du collagène des vaisseaux. Ce renforcement permet d'accroître la protection de la paroi des vaisseaux. Or, il est bien connu que les plaques d'athérome se développent d'autant plus vite que la paroi des vaisseaux est abîmée. Ces procyanidols ont également une action antioxydante qui va renforcer celle de polyphénols.

La reine des prés est une plante riche en salicylés. En effet, on a mis en évidence dans la fleur fraîche, la présence d'aldéhyde salicylique et d'un salicylate de méthyle, la gaulthérine. Ces composés ont, à faible dose, un effet anticoagulant puissant. Cet effet est en fait un effet inhibiteur sur l'agrégation des plaquettes. Cet effet renforce donc l'action de l'extrait de marc de raisin puisqu'en empêchant les plaquettes de s'agréger, les salicylés contribuent à inhiber la formation des plaques d'athérome.

La reine des prés renferme également un ensemble de composés tels que des flavonoïdes ou des composés phénoliques qui viennent compléter l'action des antioxydants contenus dans le marc de raisin.

L'action des salicylés s'exerce sur plusieurs jours, ce qui permet une accumulation des effets des doses quotidiennes et complète en cela l'action des polyphénols contenus dans l'extrait de marc de raisin qui est plus brève.

La plupart des salicylés sont des composés acides qui ont une action néfaste sur l'estomac en ce qu'ils activent la sécrétion d'acide chlorhydrique. Cette hypersécrétion acide est généralement à l'origine de maux d'estomac, voire d'ulcérations dans les cas les plus graves. Or, l'extrait de marc de raisin est riche en tanins. Ces substances très astreingnantes resserrent les tissus et vont empêcher l'excès de sécrétion acide par l'estomac, dû aux salicylés et vont donc supprimer les effets secondaires des salicylés contenus dans la reine des prés. L'extrait de marc de raisin permet donc de limiter, voire d'annuler le principal effet secondaire des salicylés.

Bien que l'action préventive sur l'athérosclérose et sur le risque de thrombose soit la plus importante, la composition selon l'invention produit d'autres effets bénéfiques.

L'action anticoagulante durable des salicylés ainsi que une action de dilatation des vaisseaux, une action tonique sur le coeur entraînant une accélération de la circulation confère à la composition selon l'invention un effet préventif contre les problèmes de stase veineuse et son principal risque, la phlébite.

La composition selon l'invention possède également des effets anti-cellulitiques combinés. En effet, l'extrait de marc de raisin contient des flavonoides, des anthocyanes et tanins catéchiques qui améliorent la circulation veineuse souvent déficiente au niveau des amas graisseux de cellulite. Les flavonoïdes permettent d'augmenter les échanges cellulaires et de désinfiltrer les tissus. Les proanthocyanidols ont une action régulatrice de la formation des fibres de collagène. Les mucilages, également contenus dans l'extrait de marc de raisin jouent un rôle efficace régulateur de l'intestin, par leur effet mécanique doux. Ils emprisonnent les aliments, régulent leur arrivée dans le sang pour éviter les pics sanguins post-prandiaux et limitent ainsi la mise en réserve dans les cellules adipeuses. Ils facilitent donc la perte de poids en cas d'excès et l'effacement progressif de la cellulite et des amas graisseux emprisonnés dans les fibres collagènes. Cette action de l'extrait de marc de raisin est potentialisée par l'action diurétique de la reine des prés puisqu'elle facilite l'élimination rénale de l'eau et permet la résorption des oedèmes. Or, il est clairement établi que la cellulite est constituée par des amas graisseux qui se forment sur les cuisses mais également par une rétention hydrique dans les cellules adipeuses qui augmentent alors de volume.

L'extrait de marc de raisin contient aussi des minéraux tels que du potassium, du calcium, du magnésium, du sodium, du fer, des sulfates, du phosphore qui couvrent les besoins quotidiens de l'homme. Il contient des acides aminés, des vitamines, en particulier du groupe B et principalement la vitamine P, qui renforcent la paroi des capillaires sanguins et s'opposent aux hémorragies et aux oedèmes, mais aussi des vitamines A et C.

L'extrait de marc de raisin peut être remplacé par d'autres extraits qui ont les mêmes effets. En effet, il est possible d'utiliser de l'extrait de vin ou de l'extrait de pépins de raisin.

Quel que soit l'extrait, l'extraction se fait de préférence par cryobroyage pour éviter la dégradation des composés par la chaleur.

Préférentiellement, on utilise comme cépage le pinot noir qui contient le plus de polyphénols et notamment de resvératrol. Cependant, il est possible d'utiliser également le cabernet sauvignon de Bordeaux ou le merlot, qui sont légèrement moins riches en polyphénols. Le raisin utilisé est donc du raisin noir. L'extrait de marc de raisin est en fait le marc qui est récupéré en fond de cuve une fois que la fermentation (ou vinification) s'est produite. Ce résidu est très riche en composés.

En ce qui concerne le végétal, il convient préférentiellement d'utiliser la reine des prés et spécialement la fleur fraîche qui est riche en aldéhyde salicylique et en salicylate de méthyle. L'extrait est obtenu à partir du produit frais par cryobroyage. Cependant, selon un autre mode de réalisation, l'extrait de reine des prés est remplacé par un extrait de saule blanc. En effet, l'écorce de ce végétal est riche en dérivés salicylés, notamment le salicoside.

D'autres produits peuvent être ajoutés à la composition. En effet, il est possible d'ajouter des extraits végétaux tels que du Polygonum cuspidatum ou du ginkgo biloba, qui ont des propriétés antioxydantes. En effet, le Polygonum cuspidatum est très concentré en resvératrol comme l'extrait de marc de raisin. Le ginkgo biloba contient des flavonoïdes très actifs, en particulier des bisflavones qui sont vasodilatateurs et qui diminuent la viscosité sanguine, mais également des hétérosides de flavones et flavonols qui tous deux ont une activité antioxydante, notamment par capture de radicaux libres au niveau de la rétine et du cerveau, ralentissant l'atteinte rétinienne et la sénescence. Aussi, ces deux extraits confèrent-ils à la composition selon l'invention des propriétés d'anti-vieillissement cérébral.

Selon un autre mode de réalisation, on ajoute également de l'ail (*Alium sativum*) et/ou de l'ananas (*Ananas comosus).* L'ail contient un composé soufré, l'alliine, qui lui confère une activité hypolipémiante. En effet, il entraîne une diminution de la concentration sanguine en cholestérol LDL et en triglycérides, et une augmentation de la concentration de cholestérol HDL. Il a également un effet anti-agrégant plaquettaire dû aux ajoènes qu'il contient. Il a donc un rôle préventif de l'athérosclérose. Il a un effet antihypertenseur dû à la dilatation des vaisseaux, et améliore la circulation sanguine, spécialement artérielle. En ce qui concerne l'ananas, on utilise préférentiellement la tige riche en bromélaïne (enzyme protéolytique). Ce composé est un anti-inflammatoire puissant qui permet de résorber les oedèmes localisés. Elle évite également la montée trop rapide de l'insulinémie et par conséquent la mise en réserve des sucres sous forme de graisses. La bromélaïne absorbée diffuse dans l'organisme et fractionne les protéines anormalement sécrétées au niveau des tissus et particulièrement les fibres de collagène de la cellulite et des hématomes. Il va donc renforcer l'action anti-cellulitique de la présente composition.

Des vitamines peuvent être également ajoutées à la composition. La vitamine E (tocophérol) est un composé antioxydant très puissant qui va renforcer l'action antioxydante de la composition. La vitamine C (acide ascorbique) peut également être ajoutée pour tonifier l'organisme ou encore la vitamine PP (amide nicotinique) pour activer la respiration cellulaire. Elles sont aussi protectrices des parois internes des vaisseaux.

Selon encore un autre mode de réalisation, la composition comprend également de l'acide acétylsalicylique ou aspirine, qui a pour rôle de renforcer l'action de la reine des prés ou du saule blanc.

La composition comprend entre 100 et 400 milligrammes (mg) d'extrait de marc de raisin, d'extrait de vin ou de pépins de raisin, et préférentiellement 200 mg. Elle comprend entre 100 et 300 mg d'extrait végétal (reine des prés ou saule blanc) et préférentiellement 150 mg.

La présente composition est préférentiellement contenue dans des gélules à avaler. Le traitement préféré consiste en une seule prise quotidienne de deux gélules après le petit-déjeûner. Le traitement peut consister également en une seule prise quotidienne.

## Revendications

1. Composition phytothérapeutique ayant une action préventive des maladies cardio-vasculaires, des thromboses vasculaires et plus généralement une action anti-vieillissement, **caractérisée en ce qu'**elle comprend un extrait pris dans le groupe composé d'extrait de marc de raisin après vinification, d'extrait de vin, d'extrait de pépins de raisin et au moins un extrait d'un végétal riche en salicylés.

2. Composition phytothérapeutique selon la revendication 1, dans laquelle ledit végétal est la reine des prés (*Filipendula ulmaria*).

3. Composition phytothérapeutique selon la revendication 1, dans laquelle ledit végétal est le saule blanc (Salix *alba*).

4. Composition phytothérapeutique selon l'une des revendications précédentes, **caractérisée en ce qu'**elle comprend également de l'extrait d'ail (*Alium sativum*).

5. Composition phytothérapeutique selon l'une des revendications précédentes, **caractérisée en ce qu'**elle comprend également de l'extrait d'ananas (*Ananas comosus).*

6. Composition phytothérapeùtique selon la revendication 5, dans laquelle l'extrait d'ananas est un extrait de tige.

7. Composition phytothérapeutique selon l'une des revendications précédentes, dans laquelle la quantité d'extrait de marc de raisin est comprise entre 100 et 400 milligrammes (mg).

8. Composition phytothérapeutique selon l'une des revendications précédentes, dans laquelle la quantité de végétal est comprise entre 100 et 300 mg.

9. Composition phytothérapeutique selon l'une des revendications précédentes, **caractérisée en ce qu'**elle comprend également de l'acide acétylsalicylique.

10. Composition phytothérapeutique selon l'une des revendications précédentes, dans laquelle le cépage utilisé pour en tirer l'extrait de marc de raisin, l'extrait de vin ou l'extrait de pépins de raisin est le pinot noir.

11. Composition phytothérapeutique selon l'une des revendications précédentes, **caractérisée en ce qu'**elle comprend également des vitamines telles que la vitamine C, la vitamine E et/ou la vitamine PP.

## Patentansprüche

1. Eine phytotherapeutische Zusammensetzung, die eine prophylaktische Wirkung bei kardiovaskulären Krankheiten, bei vaskuläreren Thrombosen und allgemeiner einen Anti-Aging-Effekt aufweist, **dadurch gekennzeichnet, dass** sie einen Extrakt beinhaltet, der der Gruppe entnommen ist, die sich aus Extrakt von Weintreber nach der Weinbereitung, Weinextrakt, Traubenkernextrakt und mindestens einem Extrakt einer an Salicylaten reichen Pflanze zusammensetzt.

2. Phytotherapeutische Zusammensetzung gemäß Anspruch 1, wobei die Pflanze Mädesüß (*Filipendula ulmaria*) ist.

3. Phytotherapeutische Zusammensetzung gemäß Anspruch 1, wobei die Pflanze Silberweide (*Salix alba*) ist.

4. Phytotherapeutische Zusammensetzung gemäß einem der vorhergehenden Ansprüche, **dadurch gekennzeichnet, dass** sie ebenfalls Knoblauchextrakt (*Alium sativum*) beinhaltet.

5. Phytotherapeutische Zusammensetzung gemäß einem der vorhergehenden Ansprüche, **dadurch gekennzeichnet, dass** sie ebenfalls Ananasextrakt (*Ananas comosus*) beinhaltet.

6. Phytotherapeutische Zusammensetzung gemäß Anspruch 5, wobei der Ananasextrakt ein Stengelextrakt ist.

7. Phythotherapeutische Zusammensetzung gemäß einem der vorhergehenden Ansprüche, wobei die Menge an Weintreberextrakt zwischen 100 und 400 Milligramm (mg) beträgt.

8. Phytotherapeutische Zusammensetzung gemäß einem der vorhergehenden Ansprüche, wobei die Pflanzenmenge zwischen 100 und 300 mg beträgt.

9. Phytotherapeutische Zusammensetzung gemäß einem der vorhergehenden Ansprüche, **dadurch gekennzeichnet, dass** sie ebenfalls Acetylsalicylsäure beinhaltet.

10. Phytotherapeutische Zusammensetzung gemäß einem der vorgehenden Ansprüche, wobei die Rebsorte, die zur Gewinnung des Weintreberextrakts, des Weinextrakts oder des Traubenkernextrakts daraus verwendet wird, Pinot Noir ist.

11. Phytotherapeutische Zusammensetzung gemäß einem der vorhergehenden Ansprüche, **dadurch gekennzeichnet, dass** sie ebenfalls Vitamine wie Vitamin C, Vitamin E und/oder Vitamin PP beinhaltet.

## Claims

1. A phytotherapeutic composition with prophylactic efficacy against cardiovascular disease and thrombosis and, more generally activity against aging, **characterized in that** it includes an extract prepared from the group composed of an extract of grape-cake left after the making of wine, a wine extract and an extract of grape seed, together with at least one extract of a plant rich in salicylates.

2. A phytotherapeutic composition according to Claim 1, in which said plant is meadowsweet (*Filipendula ulmaria*).

3. A phytotherapeutic composition according to Claim 1, in which said plant is white willow (*Salix alba*).

4. A phytotherapeutic composition according to any of the preceding Claims, **characterized in that** it also includes an extract of garlic (*Alium sativum*).

5. A phytotherapeutic composition according to any of the preceding Claims, **characterized in that** it also includes an extract of pineapple (*Ananas comosus*).

6. A phytotherapeutic composition according to Claim 5, in which the pineapple extract is prepared from stalk material.

7. A phytotherapeutic composition according to any of the preceding Claims, in which the amount of grape-cake extract is between 100 and 400 milligrams (mg).

8. A phytotherapeutic composition according to any of the preceding Claims, in which the amount of plant is between 100 and 300 mg.

9. A phytotherapeutic composition according to any of the preceding Claims, **characterized in that** it also includes acetylsalicylic acid.

10. A phytotherapeutic composition according to any of the preceding Claims, in which the vine variety used to prepare the grape-cake extract, the wine extract or the grape seed extract is Pinot Noir.

11. A phytotherapeutic composition according to any of the preceding Claims, **characterized in that** it also includes vitamins such as vitamin C, vitamin E and/or vitamin PP.
